(19) Europäisches Patentamt
European Patent Office
Office européen des brevets

(11) EP 3 586 884 A1

(12) EUROPEAN PATENT APPLICATION

(43) Date of publication:
**01.01.2020 Bulletin 2020/01**

(21) Application number: **18180068.1**

(22) Date of filing: **27.06.2018**

(51) Int Cl.:
***A61L 27/06*** *(2006.01)* ***A61L 27/18*** *(2006.01)*
***A61L 27/30*** *(2006.01)* ***A61L 27/44*** *(2006.01)*
***A61L 27/50*** *(2006.01)* ***A61N 1/375*** *(2006.01)*
***A61L 31/06*** *(2006.01)* ***A61L 31/14*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **SABIC Global Technologies B.V.**
**4612 PX Bergen op Zoom (NL)**

(72) Inventors:
- **POLO MARTINEZ, Pedro Cristobal**
  **4611 KC Bergen op Zoom (NL)**
- **DE BROUWER, Johannes**
  **5061 AT Oisterwijk (NL)**

(74) Representative: **Elzaburu S.L.P.**
**Miguel Angel 21, 2nd floor**
**28010 Madrid (ES)**

# (54) THERMOPLASTIC IMPLANT MATERIALS

(57) Devices prepared from thermoplastic resins and metals or conductive additives are disclosed. In one aspect, the article can be a medical device configured for use in a body.

EP 3 586 884 A1

## Description

### TECHNICAL FIELD

The disclosure concerns medical devices, prepared using thermoplastic resins, particularly medical implant devices.

### BACKGROUND

For any type of medical device or article intended for contact with living tissue, the risk of foreign body reaction and of infection, among many other things, are paramount concerns. Considerations regarding the materials that make up the device can be crucial in order to avoid such adverse effects that can potentially lead to the failure of the device or can harm the health of the receiving patient.

### SUMMARY

The application of thermoplastic resins in the medical field increasingly requires compositions able to the meet both the stringent physical requirements of typical polymer characteristics such as flow mechanical strength, as well biomedical considerations such as biocompatibility and stability. Accordingly, the materials selected to form the device are desired to exhibit certain characteristics, particularly biocompatibility and stability. It is understood that not all thermoplastics are suitable for implantable devices. Although certain grades of thermoplastics may be compatible, it is further understood that only suitable thermoplastics, per manufacture's guidelines or other safety recommendations, should actually be implanted, in practice.

In an aspect, the present disclosure provides thermoplastic materials appropriate for use devices such as medical devices. At least a portion of the medical device may be formed from the thermoplastic material comprising an implant grade thermoplastic resin and a metal filler or a conductive additive.

In another aspect, the disclosure concerns an article prepared according to the methods of forming a thermoplastic material comprising an implant grade thermoplastic resin and a metal coating as disclosed herein. The implant grade thermoplastic resin may qualify as a biocompatible material under ISO 10993/USP6 and may be produced under good manufacturing practices (GMP).

In one aspect, the present disclosure provides thermoplastic materials comprising an implant grade thermoplastic resin and a metal coating and/or a conductive additive for use in medical devices such as an implantable medical device.

In an aspect, the medical device can comprise a header, a body, leads, and one or more interior spacers.

### BRIEF DESCRIPTION OF THE FIGURES

FIG. 1 is a drawing of a medical device in accordance with an exemplary aspect of present disclosure.

### DETAILED DESCRIPTION

Before the present methods and devices are disclosed and described, it is to be understood that the methods and devices are not limited to specific synthetic methods, specific components, or to particular compositions. It is also to be understood that the terminology used herein is for the purpose of describing particular embodiments only and is not intended to be limiting.

Medical devices as disclosed herein can include the use of polymer compositions and thermoplastic resins. The use of thermoplastic resins and polymer compositions in medical devices requires a balance of preserving structural integrity and ensuring biocompatibility with living tissue. Implantable medical devices are implanted into the body for various reasons including orthopedic applications (e.g., hip replacement, spinal procedures, knee replacement, bone fracture repair, etc). In view of the structural integrity required by such devices, materials of fabrication are limited and generally consist of metal, plastic and composites. Metal-based conventional devices may be expensive to produce and often have limited flexibility. Thermoplastic resins of the present disclosure can provide materials suitable for use in medical devices and articles. The disclosed devices combine implant grade thermoplastic with metals and/or conductive additives, to provide a resilient, but flexible, thermoplastic implant material. A number of suitable methods may be employed to provide the disclosed thermoplastic implant materials. In some aspects, the thermoplastic resin is coated with a suitable metal. For example, an electroplating process or a deposition process, such as vapor deposition, may be used to combine the thermoplastic resin and a suitable metal. In yet further aspects, electroplating processes may be used. In further aspects, the thermoplastic resin may be combined with a conductive filler.

## POLYMER COMPOSITION

In one aspect of the disclosure, the medical device formed may be formed using a polymer composition. In one aspect of the present disclosure, the polymer composition can comprise a thermoplastic resin. Other components, however, may also be included in the thermoplastic resin. For example, the polymer composition may also include a ceramic and a metal.

In one aspect of the disclosure, the polymer composition can be suitable for melt processing such that the medical device, or a component thereof, can be formed using a melt process and in particular, additive manufacturing or injection molding. The polymer composition can include any polymeric material known in the art. The polymer composition may be composed of more than one polymeric material.

In one aspect of the disclosure, the polymers used in the polymer composition may be selected from a wide variety of thermoplastic polymers, and blends of thermoplastic polymers. The polymer composition can comprise a homopolymer, a copolymer such as a star block copolymer, a graft copolymer, an alternating block copolymer or a random copolymer, ionomer, dendrimer, or a combination comprising at least one of the foregoing. The polymer composition may also be a blend of polymers, copolymers, terpolymers, or the like, or a combination comprising at least one of the foregoing.

Examples of thermoplastic polymers that can be used in the polymer composition include polyacetals, polyacrylics, polycarbonates, polyalkyls, polystyrenes, polyolefins, polyesters, polyamides, polyaramides, polyamide-imides, polyarylates, polyurethanes, epoxies, phenolics, silicones, polyarylsulfones, polyethersulfones, polyphenylene sulfides, polysulfones, polyimides, polyetherimides, polytetrafluoroethylenes, polyetherketones, polyether ether ketones, polyether ketone ketones, polybenzoxazoles, polyoxadiazoles, polybenzothiazinophenothiazines, polybenzothiazoles, polypyrazinoquinoxalines, polypyromellitimides, polyquinoxalines, polybenzimidazoles, polyoxindoles, polyoxoisoindolines, polydioxoisoindolines, polytriazines, polypyridazines, polypiperazines, polypyridines, polypiperidines, polytriazoles, polypyrazoles, polycarboranes, polyoxabicyclononanes, polydibenzofurans, polyphthalides, polyacetals, polyanhydrides, polyvinyl ethers, polyvinyl thioethers, polyvinyl alcohols, polyvinyl ketones, polyvinyl halides, polyvinyl nitriles, polyvinyl esters, polysulfonates, polysulfides, polythioesters, polysulfonamides, polyureas, polyphosphazenes, polysilazanes, polypropylenes, polyethylenes, polyethylene terephthalates, polyvinylidene fluorides, polysiloxanes, or the like, or a combination comprising at least one of the foregoing thermoplastic polymers.

Examples of blends of thermoplastic polymers that can be used polymer composition resin include acrylonitrile-butadiene-styrene/nylon, polycarbonate/acrylonitrile-butadiene-styrene, polyphenylene ether/polystyrene, polyphenylene ether/polyamide, polycarbonate/polyester, polyphenylene ether/polyolefin, or the like, or a combination comprising at least one of the foregoing.

In various examples, implant grade thermoplastics of the present disclosure may comprise polyimides such as polyetherimide PEI, polysulfone PSU, polyphenylsulfone PPSU, polyether ether ketone PEEK, or polyphthalamide PPA, or a combination thereof. In a further aspect, polyimides used in the disclosed thermoplastic resin may include polyamide-imides, polyetherimides and polybenzimidazoles. In a further aspect, polyetherimides comprise melt processable polyetherimides. Any of the foregoing thermoplastic resins may be regarded as implantable should the resin meet applicable standards such as those according to ASTM or ISO. However, it is understood that such implantation should consider manufacturer and safety guidelines. In one example, the thermoplastic resin may be useful as an implant grade thermoplastic resin where the resin qualifies as a biocompatible material under ISO 10993/USP6. Resins that meet these standards may be appropriate for implantation in tissue.

## POLYETHERIMIDE

In one aspect of the present disclosure, a medical device, or a component thereof, can be formed from a polyetherimide resin. Polyetherimides ("PEIs") are amorphous, transparent, high performance polymers having a glass transition temperature ("Tg") of greater than 180 °C. PEIs further have high strength, heat resistance, and modulus, and broad chemical resistance. The high reliability and safety benefits afforded by a polyetherimide from its optical transparency, toughness, and heat resistance can be useful in medical applications.

In an aspect, polyetherimides can comprise polyetherimides homopolymers (e.g., polyetherimidesulfones) and polyetherimides copolymers. The polyetherimide can be selected from (i) polyetherimidehomopolymers, e.g., polyetherimides, (ii) polyetherimide co-polymers, and (iii) combinations thereof. Polyetherimides are known polymers and are sold by SABIC Innovative Plastics under the ULTEM™*, EXTEM™*, and Siltem* brands (Trademark of SABIC).

In an aspect, the polyetherimides can be of formula (1):

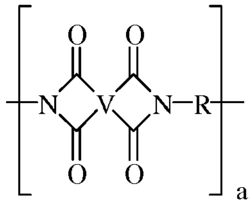

(1),

wherein a is more than 1, for example 10 to 1,000 or more, or more specifically 10 to 500.

The group V in formula (1) is a tetravalent linker containing an ether group (a "polyetherimide" as used herein) or a combination of an ether groups and arylenesulfone groups (a "polyetherimidesulfone"). Such linkers include but are not limited to: (a) substituted or unsubstituted, saturated, unsaturated or aromatic monocyclic and polycyclic groups having 5 to 50 carbon atoms, optionally substituted with ether groups, arylenesulfone groups, or a combination of ether groups and arylenesulfone groups; and (b) substituted or unsubstituted, linear or branched, saturated or unsaturated alkyl groups having 1 to 30 carbon atoms and optionally substituted with ether groups or a combination of ether groups, arylenesulfone groups, and arylenesulfone groups; or combinations comprising at least one of the foregoing. Suitable additional substitutions include, but are not limited to, ethers, amides, esters, and combinations comprising at least one of the foregoing.

The R group in formula (1) includes but is not limited to substituted or unsubstituted divalent organic groups such as: (a) aromatic hydrocarbon groups having 6 to 20 carbon atoms and halogenated derivatives thereof; (b) straight or branched chain alkylene groups having 2 to 20 carbon atoms; (c) cycloalkylene groups having 3 to 20 carbon atoms, or (d) divalent groups of formula (2):

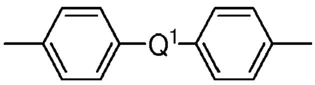

(2),

wherein Q1 includes but is not limited to a divalent moiety such as -O-, -S-, -C(O)-, -SO2-, -SO-, -CyH2y- (y being an integer from 1 to 5), and halogenated derivatives thereof, including perfluoroalkylene groups.

In an embodiment, linkers V include but are not limited to tetravalent aromatic groups of formula (3):

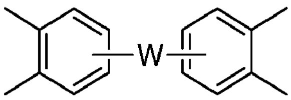

(3),

wherein W is a divalent moiety including -O-, -SO2-, or a group of the formula -O-Z-O-wherein the divalent bonds of the -O- or the -O-Z-O- group are in the 3,3', 3,4', 4,3', or the 4,4' positions, and wherein Z includes, but is not limited, to divalent groups of formulas (4):

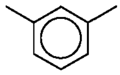 , 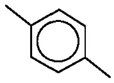 , 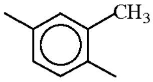 ,

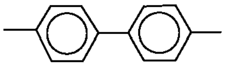 , 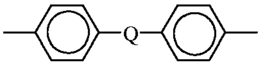 ,

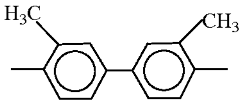 , 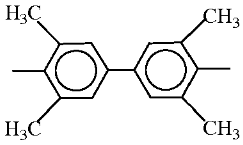 ,

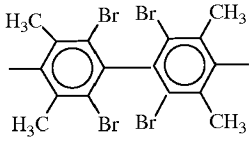 , and

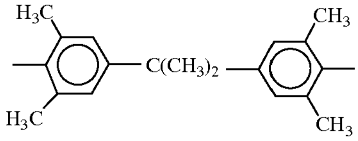 (4),

wherein Q includes, but is not limited to a divalent moiety including -O-, -S-, -C(O), $-SO_2-$, -SO-, $-C_yH_{2y}-$ (y being an integer from 1 to 5), and halogenated derivatives thereof, including perfluoroalkylene groups.

In an aspect, the polyetherimide comprise more than 1, specifically 10 to 1,000, or more specifically, 10 to 500 structural units, of formula (5):

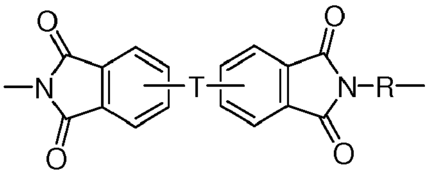 (5),

wherein T is -O- or a group of the formula -O-Z-O- wherein the divalent bonds of the -O-or the -O-Z-O- group are in the 3,3', 3,4', 4,3', or the 4,4' positions; Z is a divalent group of formula (3) as defined above; and R is a divalent group of formula (2) as defined above.

In another aspect, the polyetherimidesulfones are polyetherimides comprising ether groups and sulfone groups wherein at least 50 mole % of the linkers V and the groups R in formula (1) comprise a divalent arylenesulfone group. For example, all linkers V, but no groups R, can contain an arylenesulfone group; or all groups R but no linkers V can contain an arylenesulfone group; or an arylenesulfone can be present in some fraction of the linkers V and R groups, provided that the total mole fraction of V and R groups containing an aryl sulfone group is greater than or equal to 50 mole%.

Even more specifically, polyetherimidesulfones can comprise more than 1, specifically 10 to 1,000, or more specifically, 10 to 500 structural units of formula (6):

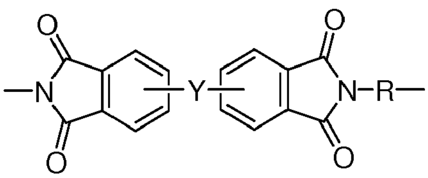 (6),

wherein Y is -O-, -SO2-, or a group of the formula -O-Z-O- wherein the divalent bonds of the -O-, SO2-, or the -O-Z-O- group are in the 3,3', 3,4', 4,3', or the 4,4' positions, wherein Z is a divalent group of formula (3) as defined above and R is a divalent group of formula (2) as defined above, provided that greater than 50 mole% of the sum of moles Y + moles R in formula (2) contain -SO2- groups.

It is to be understood that the polyetherimides and polyetherimidesulfones can optionally comprise linkers V

that do not contain ether or ether and sulfone groups, for example linkers of formula (7):

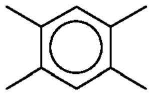
,
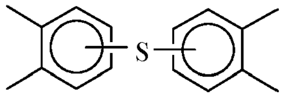
, and
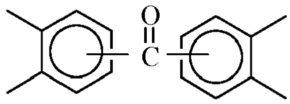
(7).

Imide units containing such linkers are generally be present in amounts ranging from 0 to 10 mole % of the total number of units, specifically 0 to 5 mole %. In one embodiment no additional linkers V are present in the polyetherimides.

In another aspect, the polyetherimide comprises 10 to 500 structural units of formula (5) and the polyetherimidesulfone contains 10 to 500 structural units of formula (6).

Polyetherimides can be prepared by any suitable process. In one embodiment, polyetherimides and polyetherimide copolymers include polycondensation polymerization processes and halo-displacement polymerization processes.

Polycondensation methods can include a method for the preparation of polyetherimides having structure (1) is referred to as the nitro-displacement process (X is nitro in formula (8)). In one example of the nitro-displacement process, N-methyl phthalimide is nitrated with 99% nitric acid to yield a mixture of N-methyl-4-nitrophthalimide (4-NPI) and N-methyl-3-nitrophthalimide (3-NPI). After purification, the mixture, containing approximately 95 parts of 4-NPI and 5 parts of 3-NPI, is reacted in toluene with the disodium salt of bisphenol-A (BPA) in the presence of a phase transfer catalyst. This reaction yields BPA-bisimide and $NaNO_2$ in what is known as the nitro-displacement step. After purification, the BPA-bisimide is reacted with phthalic anhydride in an imide exchange reaction to afford BPA-dianhydride (BPADA), which in turn is reacted with a diamine such as meta-phenylene diamine (MPD) in ortho-dichlorobenzene in an imidization-polymerization step to afford the product polyetherimide.

Other diamines are also possible. Examples of suitable diamines include: m-phenylenediamine; p-phenylenediamine; 2,4-diaminotoluene; 2,6-diaminotoluene; m-xylylenediamine; p-xylylenediamine; benzidine; 3,3'-dimethylbenzidine; 3,3'-dimethoxybenzidine; 1,5-diaminonaphthalene; bis(4-aminophenyl)methane; bis(4-aminophenyl)propane; bis(4-aminophenyl)sulfide; bis(4-aminophenyl)sulfone; bis(4-aminophenyl)ether; 4,4'-diaminodiphenylpropane; 4,4'-diaminodiphenylmethane(4,4'-methylenedianiline); 4,4'-diaminodiphenylsulfide; 4,4'-diaminodiphenylsulfone; 4,4'-diaminodiphenylether(4,4'-oxydianiline); 1,5-diaminonaphthalene; 3,3'dimethylbenzidine; 3-methylheptamethylenediamine; 4,4-dimethylheptamethylenediamine; 2,2',3,3'-tetrahydro-3,3,3',3'-tetramethyl-1,1'-spirobi[1H-indene]-6,6'-diamine; 3,3',4,4'-tetrahydro-4,4,4',4'-tetramethyl-2,2'-spirobi[2H-1-benzo- pyran]-7,7'-diamine; 1,1'-bis[1-amino-2-methyl-4-phenyl]cyclohexane, and isomers thereof as well as mixtures and blends comprising at least one of the foregoing. In one embodiment, the diamines are specifically aromatic diamines, especially m-and p-phenylenediamine and mixtures comprising at least one of the foregoing.

Suitable dianhydrides that can be used with the diamines include and are not limited to 2,2-bis[4-(3,4-dicarboxyphenoxy)phenyl]propane dianhydride; 4,4'-bis(3,4-dicarboxyphenoxy)diphenyletherdianhydride; 4,4'-bis(3,4-dicarboxyphenoxy)diphenylsulfidedianhydride; 4,4'-bis(3,4-dicarboxyphenoxy)benzophenonedianhydride; 4,4'-bis(3,4-dicarboxyphenoxy)diphenylsulfonedianhydride; 2,2-bis[4-(2,3-dicarboxyphenoxy)phenyl]propane dianhydride; 4,4'-bis(2,3-dicarboxyphenoxy)diphenyletherdianhydride; 4,4'-bis(2,3-dicarboxyphenoxy)diphenylsulfidedianhydride; 4,4'-bis(2,3-dicarboxyphenoxy)benzophenonedianhydride; 4,4'-bis(2,3-dicarboxyphenoxy)diphenylsulfonedianhydride; 4-(2,3-dicarboxyphenoxy)-4'-(3,4-dicarboxyphenoxy)diphenyl-2,2-propane dianhydride; 4-(2,3-dicarboxyphenoxy)-4'-(3,4-dicarboxyphenoxy)diphenyletherdianhydride; 4-(2,3-dicarboxyphenoxy)-4'-(3,4-dicarboxyphenoxy)diphenylsulfide dianhydride; 4-(2,3-dicarboxyphenoxy)-4'-(3,4-dicarboxyphenoxy)benzophenonedianhydride; 4-(2,3-dicarboxyphenoxy)-4'-(3,4-dicarboxyphenoxy)diphenylsulfone dianhydride; 1,3-bis(2,3-dicarboxyphenoxy)benzene dianhydride; 1,4-bis(2,3-dicarboxyphenoxy)benzene dianhydride; 1,3-bis(3,4-dicarboxyphenoxy)benzene dianhydride; 1,4-bis(3,4-dicarboxyphenoxy)benzene dianhydride; 3,3',4,4'-diphenyl tetracarboxylicdianhydride; 3,3',4,4'-benzophenonetetracarboxylic dianhydride; naphthalicdianhydrides, such as 2,3,6,7-naphthalic dianhydride, etc.; 3,3',4,4'-biphenylsulphonictetracarboxylic dianhydride; 3,3',4,4'-biphenylethertetracarboxylic dianhydride; 3,3',4,4'-dimethyldiphenylsilanetetracarboxylic dianhydride; 4,4'-bis (3,4-dicarboxyphenoxy)diphenylsulfidedianhydride; 4,4'-bis (3,4-dicarboxyphenoxy)diphenylsulphonedianhydride; 4,4'-bis (3,4-dicarboxyphenoxy)diphenylpropanedianhydride; 3,3',4,4'-biphenyltetracarboxylic dianhydride; bis(phthalic)phenylsulphineoxidedianhydride; p-phenylene-bis(triphenylphthalic)dianhydride; m-phenylene-bis(triphenylphthalic)dianhydride; bis(triphenylphthalic)-4,4'-diphenylether dianhydride; bis(triphenylphthalic)-4,4'-diphenylmethane dianhydride; 2,2'-bis(3,4-dicarboxyphenyl)hexafluoropropanedianhydride; 4,4'-oxydiphthalic dianhydride; pyromelliticdianhydride; 3,3',4,4'-diphenylsulfonetetracarboxylic dianhydride; 4',4'-bisphenol A dianhydride; hydroquinone diphthalic dianhydride; 6,6'-bis(3,4-dicarboxyphenoxy)-2,2',3,3'-tetrahydro-3,3,3',3'-tetramethyl- -1,1'-spirobi[1H-indene]dianhydride; 7,7'-bis(3,4-dicarboxyphenoxy)-3,3',4,4'-tetrahydro-4,4,4',4'-tetramethyl- -2,2'-spirobi[2H-1-benzopyran]dianhydride; 1,1'-bis[1-(3,4-dicarboxyphenoxy)-2-methyl-4-phenyl]cyclohexane dianhydride; 3,3',4,4'-diphenylsulfonetetracarboxylic dianhydride; 3,3',4,4'-diphenylsulfidetetracarboxylic dianhydride; 3,3',4,4'-diphenylsulfoxidetetracarboxylic dianhydride; 4,4'-oxydiphthalic dianhydride; 3,4'-oxydiphthalic dianhydride; 3,3'-oxydiphthalic dianhydride; 3,3'-benzophenonetetracarboxylic dianhydride; 4,4'-carbonyldiphthalic dianhydride; 3,3',4,4'-diphenylmethanetetracarboxylic dianhydride; 2,2-bis(4-(3,3-dicarboxyphenyl)propane dianhydride; 2,2-bis(4-(3,3-dicarboxyphenyl)hexafluoropropanedianhydride; (3,3',4,4'-diphenyl)phenylphosphinetetracarboxylicdianhydride; (3,3',4,4'-diphenyl)phenylphosphineoxidetetracarboxylicdianhydride; 2,2'-dichloro-3,3',4,4'-biphenyltetracarboxylic dianhydride; 2,2'-dimethyl-3,3',4,4'-biphenyltetracarboxylic dianhydride; 2,2'-dicyano-3,3',4,4'-biphenyltetracarboxylic dianhydride; 2,2'-dibromo-3,3',4,4'-biphenyltetracarboxylic dianhydride; 2,2'-diiodo-3,3',4,4'-biphenyltetracarboxylic dianhydride; 2,2'-ditrifluoromethyl-3,3',4,4'-biphenyltetracarboxylic dianhydride; 2,2'-bis(1-methyl-4-phenyl)-3,3',4,4'-biphenyltetracarboxylic dianhydride; 2,2'-bis(1-trifluoromethyl-2-phenyl)-3,3',4,4'-biphenyltetracarboxylic dianhydride; 2,2'-bis(1-trifluoromethyl-3-phenyl)-3,3',4,4'-biphenyltetracarboxylic dianhydride; 2,2'-bis(1-trifluoromethyl-4-phenyl)-3,3',4,4'-biphenyltetracarboxylic dianhydride; 2,2'-bis(1-phenyl-4-phenyl)-3,3',4,4'-biphenyltetracarboxylic dianhydride; 4,4'-bisphenol A dianhydride; 3,4'-bisphenol A dianhydride; 3,3'-bisphenol A dianhydride; 3,3',4,4'-diphenylsulfoxidetetracarboxylic dianhydride; 4,4'-carbonyldiphthalic dianhydride; 3,3',4,4'-diphenylmethanetetracarboxylic dianhydride; 2,2'-bis(1,3-trifluoromethyl-4-phenyl)-3,3',4,4'-biphenyltetracarboxylic dianhydride, and all isomers thereof, as well as combinations of the foregoing.

Halo-displacement polymerization methods for making polyetherimides and polyetherimidesulfones include and are not limited to, the reaction of a bis(phthalimide) for formula (8):

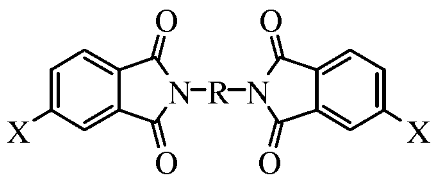

(8),

wherein R is as described above and X is a nitro group or a halogen. Bis-phthalimides (8) can be formed, for example, by the condensation of the corresponding anhydride of formula (9):

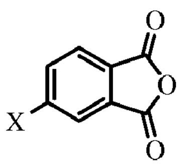

(9),

wherein X is a nitro group or halogen, with an organic diamine of the formula (10):

$$H_2N\text{-}R\text{-}NH_2 \qquad (10),$$

wherein R is as described above.

Illustrative examples of amine compounds of formula (10) include: ethylenediamine, propylenediamine, trimethylenediamine, diethylenetriamine, triethylenetetramine, hexamethylenediamine, heptamethylenediamine, octamethylenediamine, nonamethylenediamine, decamethylenediamine, 1,12-dodecanediamine, 1,18-octadecanediamine, 3-methylheptamethylenediamine, 4,4-dimethylheptamethylenediamine, 4-methylnonamethylenediamine, 5-methylnonamethylenediamine, 2,5-dimethylhexamethylenediamine, 2,5-dimethylheptamethylenediamine, 2, 2-dimethylpropylenediamine, N-methyl-bis (3-aminopropyl) amine, 3-methoxyhexamethylenediamine, 1,2-bis(3-aminopropoxy) ethane, bis(3-aminopropyl) sulfide, 1,4-cyclohexanediamine, bis-(4-aminocyclohexyl) methane, m-phenylenediamine, p-phenylenediamine, 2,4-diaminotoluene, 2,6-diaminotoluene, m-xylylenediamine, p-xylylenediamine, 2-methyl-4,6-diethyl-1,3-phenylene-diamine, 5-methyl-4,6-diethyl-1,3-phenylene-diamine, benzidine, 3,3'-dimethylbenzidine, 3,3'-dimethoxybenzidine, 1,5-diaminonaphthalene, bis(4-aminophenyl) methane, bis(2-chloro-4-amino-3, 5-diethylphenyl) methane, bis(4-aminophenyl) propane, 2,4-bis(b-amino-t-butyl) toluene, bis(p-b-amino-t-butylphenyl) ether, bis(p-b-methyl-o-aminophenyl) benzene, bis(p-b-methyl-o-aminopentyl) benzene, 1, 3-diamino-4-isopropylbenzene, bis(4-aminophenyl) ether and 1,3-bis(3-aminopropyl) tetramethyldisiloxane. Mixtures of these amines can be used. Illustrative examples of amine compounds of formula (10) containing sulfone groups include but are not limited to, diaminodiphenylsulfone (DDS) and bis(aminophenoxy phenyl) sulfones (BAPS). Combinations comprising any of the foregoing amines can be used.

The polyetherimides can be synthesized by the reaction of the bis(phthalimide) (8) with an alkali metal salt of a dihydroxy substituted aromatic hydrocarbon of the formula HO-V-OH wherein V is as described above, in the presence or absence of phase transfer catalyst. Suitable phase transfer catalysts are disclosed in U.S. Patent No. 5,229,482. Specifically, the dihydroxy substituted aromatic hydrocarbon a bisphenol such as bisphenol A, or a combination of an alkali metal salt of a bisphenol and an alkali metal salt of another dihydroxy substituted aromatic hydrocarbon can be used.

In one embodiment, the polyetherimide comprises structural units of formula (5) wherein each R is independently p-phenylene or m-phenylene or a mixture comprising at least one of the foregoing; and T is group of the formula -O-Z-O- wherein the divalent bonds of the -O-Z-O- group are in the 3,3' positions, and Z is 2,2-diphenylenepropane group (a bisphenol A group). Further, the polyetherimidesulfone comprises structural units of formula (6) wherein at least 50 mole% of the R groups are of formula (4) wherein Q is - SO2- and the remaining R groups are independently p-phenylene or m-phenylene or a combination comprising at least one of the foregoing; and T is group of the formula -O-Z-O- wherein the divalent bonds of the -O-Z-O- group are in the 3,3' positions, and Z is a 2,2-diphenylenepropane group.

The polyetherimide and polyetherimidesulfone can be used alone or in combination with each other and/or other of the disclosed polymeric materials in fabricating the polymeric components of the invention. In one embodiment, only the polyetherimide is used. In another embodiment, the weight ratio of polyetherimide: polyetherimidesulfone can be from 99:1 to 50:50.

The polyetherimides can have a weight average molecular weight (Mw) of 5,000 to 100,000 grams per mole (g/mole) as measured by gel permeation chromatography (GPC). In some embodiments the Mw can be 10,000 to 80,000. The molecular weights as used herein refer to the absolute weight averaged molecular weight (Mw).

The polyetherimides can have an intrinsic viscosity greater than or equal to 0.2 deciliters per gram (dl/g) as measured in m-cresol at 25°C. Within this range the intrinsic viscosity can be 0.35 to 1.0 dl/g, as measured in m-cresol at 25°C.

The polyetherimides can have a glass transition temperature of greater than 180°C, specifically of 200°C to 500°C, as measured using differential scanning calorimetry (DSC) per ASTM test D3418. In some embodiments, the polyetherimide and, in particular, a polyetherimide has a glass transition temperature of 240 to 350°C.

The polyetherimides can have a melt index of 0.1 to 10 grams per minute (g/min), as measured by American Society for Testing Materials (ASTM) DI 238 at 340 to 370° C., using a 6.7 kilogram (kg) weight.

In certain aspects, the polyetherimides of the present disclosure may be unfilled, standard flow grades (PEI-1 in Tables 1-2) or unfilled, high flow grades (PEI-2 in Tables 1-2), or may be filled, for example, with carbon (e.g., carbon fiber) or glass. Filled polymer components may include between 40 wt% and 90 wt% of the polyetherimide resin and between 10 wt% and 60 wt% of a filler by weight of the polymer component. Other formulations may be used.

An alternative halo-displacement polymerization process for making polyetherimides, e.g., polyetherimides having structure (1) is a process referred to as the chloro-displacement process (X is Cl in formula (8)). The chloro-displacement process is illustrated as follows: 4-chloro phthalic anhydride and meta-phenylene diamine are reacted in the presence of a catalytic amount of sodium phenyl phosphinate catalyst to produce the bischlorophthalimide of meta-phenylene diamine (CAS No. 148935-94-8). The bischlorophthalimide is then subjected to polymerization by chloro-displacement reaction with the disodium salt of BPA in the presence of a catalyst in ortho-dichlorobenzene or anisole solvent. Alternatively, mixtures of 3-chloro- and 4-chlorophthalic anhydride may be employed to provide a mixture of isomeric bischlorophthalimides which may be polymerized by chloro-displacement with BPA disodium salt as described above.

Siloxane polyetherimides can include polysiloxane/polyetherimide block or random copolymers having a siloxane content of greater than 0 and less than 40 weight percent (wt%) based on the total weight of the block copolymer. The block copolymer comprises a siloxane block of Formula (I):

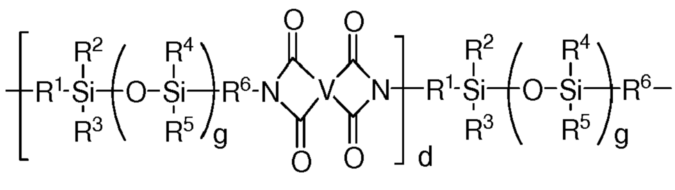

(I);

wherein $R^{1-6}$ are independently at each occurrence selected from the group consisting of substituted or unsubstituted, saturated, unsaturated, or aromatic monocyclic groups having 5 to 30 carbon atoms, substituted or unsubstituted, saturated, unsaturated, or aromatic polycyclic groups having 5 to 30 carbon atoms, substituted or unsubstituted alkyl groups

having 1 to 30 carbon atoms and substituted or unsubstituted alkenyl groups having 2 to 30 carbon atoms, V is a tetravalent linker selected from the group consisting of substituted or unsubstituted, saturated, unsaturated, or aromatic monocyclic and polycyclic groups having 5 to 50 carbon atoms, substituted or unsubstituted alkyl groups having 1 to 30 carbon atoms, substituted or unsubstituted alkenyl groups having 2 to 30 carbon atoms and combinations comprising at least one of the foregoing linkers, g equals 1 to 30, and d is 2 to 20. Commercially available siloxane polyetherimides can be obtained from SABIC Innovative Plastics under the brand name SILTEM* (*Trademark of SABIC Innovative Plastics IP B.V.)

The polyetherimide resin can have a weight average molecular weight (Mw) within a range having a lower limit and/or an upper limit. The range can include or exclude the lower limit and/or the upper limit. The lower limit and/or upper limit can be selected from 5000, 6000, 7000, 8000, 9000, 10000, 11000, 12000, 13000, 14000, 15000, 16000, 17000, 18000, 19000, 20000, 21000, 22000, 23000, 24000, 25000, 26000, 27000, 28000, 29000, 30000, 31000, 32000, 33000, 34000, 35000, 36000, 37000, 38000, 39000, 40000, 41000, 42000, 43000, 44000, 45000, 46000, 47000, 48000, 49000, 50000, 51000, 52000, 53000, 54000, 55000, 56000, 57000, 58000, 59000, 60000, 61000, 62000, 63000, 64000, 65000, 66000, 67000, 68000, 69000, 70000, 71000, 72000, 73000, 74000, 75000, 76000, 77000, 78000, 79000, 80000, 81000, 82000, 83000, 84000, 85000, 86000, 87000, 88000, 89000, 90000, 91000, 92000, 93000, 94000, 95000, 96000, 97000, 98000, 99000, 100000, 101000, 102000, 103000, 104000, 105000, 106000, 107000, 108000, 109000, and 110000 Daltons. For example, the polyetherimide resin can have a weight average molecular weight (Mw) from 5,000 to 100,000 Daltons, from 5,000 to 80,000 Daltons, or from 5,000 to 70,000 Daltons. The primary alkyl amine modified polyetherimide will have lower molecular weight and higher melt flow than the starting, unmodified, polyetherimide.

The polyetherimide resin can be selected from the group consisting of a polyetherimide, for example as described in US patents 3,875,116; 6,919,422 and 6,355,723 a silicone polyetherimide, for example as described in US patents 4,690,997; 4,808,686 a polyetherimidesulfone resin, as described in US patent 7,041,773 and combinations thereof, each of these patents are incorporated herein their entirety.

The polyetherimide resin can have a glass transition temperature within a range having a lower limit and/or an upper limit. The range can include or exclude the lower limit and/or the upper limit. The lower limit and/or upper limit can be selected from 100, 110, 120, 130, 140, 150, 160, 170, 180, 190, 200, 210, 220, 230, 240, 250, 260, 270, 280, 290, 300 and 310 degrees Celsius. For example, the polyetherimide resin can have a glass transition temperature (Tg) greater than about 200 degrees Celsius.

The polyetherimide resin can be substantially free (less than 100 parts per million, ppm) of benzylic protons. The polyetherimide resin can be free of benzylic protons. The polyetherimide resin can have an amount of benzylic protons below 100 ppm. In one embodiment, the amount of benzylic protons ranges from more than 0 to below 100 ppm. In another embodiment, the amount of benzylic protons is not detectable.

The polyetherimide resin can be substantially free (less than 100 ppm) of halogen atoms. The polyetherimide resin can be free of halogen atoms. The polyetherimide resin can have an amount of halogen atoms below 100 ppm. In one embodiment, the amount of halogen atoms range from more than 0 to below 100 ppm. In another embodiment, the amount of halogen atoms is not detectable.

In various aspects, the polyetherimide polymer can be used in a number of forms. As an example, the polyetherimide resin can comprise continuous polymeric filaments or fibers. The polyetherimide resin can comprise fibers having a diameter of from about 0.00001 millimeters to about 2 millimeters (mm). In further examples, the polyetherimide resin can comprise sheets.

## METAL COATING

The present disclosure provides thermoplastic implant materials comprising a thermoplastic resin and a suitable metal filler and/or suitable conductive material. Suitable metals may include, but are not limited to certain Group III and Group IV metals, certain Group IB to VIIIB transition metals, and certain metalloids. In some examples, the metal may comprise gold, lead, cadmium, mercury, iridium, tantalum, stainless steel, cobalt, or a combination (such as an alloy) thereof. In further examples, the metal may comprise a transition metal. For example, the metal may comprise titanium or zirconium. The thermoplastic implant device may comprise a metal or a metal alloy. It is noted that certain metals described herein may be in an oxidized form. These oxidized metals, also described as metal oxides, may also be suitable metal coatings for the present disclosure. For example, zirconium oxide and titanium oxide may comprise suitable metal coatings. The metal coating may be introduced to a desired thermoplastic resin according to a number of processes. In further examples, a metal may be coated or deposited at a surface of a desired thermoplastic resin. Methods of preparing the thermoplastic resin with a metal coating are disclosed herein.

## CONDUCTIVE ADDITIVE

The thermoplastic implant device may comprise a conductive additive. The conductive additive may comprise

a conductive carbon compound. A conductive additive may refer to an additive that is electrically or thermally conductive, particularly electrically conductive. In some examples, an electrically conductive additive may have a conductivity of at least $1\times10^3$ Siemens per meter when measured in solution at 20 °C. Highly thermally conductive fillers may have a thermal conductivity greater than or equal to 50 Watts per meter Kelvin W/m·K. Low thermal conductive fillers can have thermal conductivities in the range of from about 0.0001 to about 30 W/m·K, or from about 10 W/m·K to about 20 w/m·K.

Conductive carbon compounds include, but are not limited to carbon nanotubes (thermal conductivity greater than 3,000 W/m·K at room temperature RT; electrical conductivity, about $10^6$ to $10^7$ Siemens per meter), graphene (thermal conductivity greater than 1000 W/m·K at RT; electrical conductivity, greater than $10^3$ S/m S/m), graphite (thermal conductivity greater than 500 W/m·K at RT; electrical conductivity, about $2.00\times10^5$ S/m to $3.00\times10^5$ S/m $\|$basal plane, $3.30\times10^2$ $\perp$basal plane Siemens per meter), coated graphite, expanded graphite, carbon fiber, carbon black, or graphitized carbon black.

The conductive additive may be introduced to the desired thermoplastic resin used to form a medical device, or a part thereof. Methods of combining the thermoplastic resin and conductive additive are disclosed herein.

## ADDITIONAL COMPONENTS

In an aspect, the thermoplastic resin used to form the medical device, or a part thereof, can include certain additional components. In one aspect, the thermoplastic resin can include a biocide. The biocide can include germicides, antimicrobials, antibiotics, antibacterials, anti-yeasts, anti-algals, antivirals, antifungals, antiprotozoals, antiparasites, or combinations thereof.

## PROPERTIES AND ARTICLES

The present disclosure relates to articles comprising thermoplastic resins and a metal filler or conductive carbon disclosed herein. In certain aspects, the articles can be formed from polyetherimide resins and a metal filler or conductive carbon as described herein. The advantageous mechanical characteristics of the polyetherimides disclosed herein can make them appropriate for an array of articles. Suitable articles can be exemplified by, but are not limited to automotive components, including exterior and interior components; domestic appliances; enclosures for electrical and telecommunication devices; marine equipment; and medical instruments. The disclosure further contemplates additional fabrication operations on said articles.

In one aspect of the present disclosure, the article can comprise a medical device, such as an implantable medical device, or a component thereof, prepared from polyetherimide. As an example, the medical device can be used to provide diagnostics or to deliver treatment and can include one or more electrodes coupled to circuitry located on or within the device. The circuitry can be configured to monitor the electrical activity of a given organ to which it is connected. Exemplary implantable medical devices comprising polyetherimide can include cardiac rhythm management devices such as implantable pacemakers, implantable defibrillators (such as, implantable cardioverter-defibrillators (ICDSs)), cardiac resynchronization therapy devices (CRTSs), neural stimulators and modulators, or one or more other devices.

In an exemplary aspect, the implantable medical device comprising a thermoplastic resin configured to diagnose and modify cardiac function. Electrical stimulus in a normally operating heart drives the blood pumping function of the organ. The electrical stimulus is generated within the right atrium and transmitted to the ventricle where the stimulus provides a contracting or beating of the ventricle. Aging and cardiovascular disease, among other conditions, can obstruct the electrical stimulus in the heart and prevent the heart from beating at its normal rate oftentimes resulting in fatigue, severe illness, or death. As an example, an implantable medical device can be placed within the cardiac tissue to monitor the cardiac activity and to detect when electrical stimulus has been obstructed. When obstruction becomes apparent, the device can provide electrical stimulus to the heart until the organ regains the ability to effectively operate. As such, the device can utilize a power source and circuitry to deliver the electrical therapy to elicit the necessary stimuli.

In various aspects, the medical device can comprise a header, a body or enclosure, and one or more leads. FIG. 1 provides an exemplary implantable medical device 100. The medical device 100 may comprise a header 102, an enclosure 104, and one or more leads 106. The header 102 of the medical device can be coupled to the enclosure 104. The header 102 can further comprise one or more electrical contacts 108. The electrical contacts 108 can in turn be connected to one or more leads 106. The header can include one or more bores 112, 114 or openings to allow passage of the leads 106 through the header 102.

In an aspect, the header 102 can be coupled to the enclosure 104 to facilitate electrical communication between elements within the sealed enclosure 104 and elements external to the sealed enclosure 104. The coupling of the header 102 and the enclosure 104 can allow passage of the leads 106 from the electrical contacts 108 into the enclosure 104 to connect to the power source and circuitry situated therein.

The header 102 can be formed from a resin that is molded and cured into desired configuration. In an exemplary method, the header 102 can be molded separately from the enclosure 104. In other methods, the header 102 can be

molded while in contact with the enclosure. In one example, the header 102 can be transparent. A transparent header 102 can be useful because components such as the electrical contacts 108 can be visibly inspected.

In an aspect, the enclosure 104 can be configured to house circuitry and a power source necessary to monitor and modify cardiac function or other biological functions. The power source can comprise one or more electrochemical cells and operative electric circuitry within the enclosure 104. The enclosure of the implantable medical device can be hermetically sealed to protect the power source and circuitry components from corrosive bodily fluids.

In various aspects, a spacer member or one or more spacers 116 can be disposed within the enclosure to situate the power source comprising one or more electrochemical cells and operative electric circuitry. The spacers 116 can be formed from a thermoplastic resin. In one example, the one or more spacers can be formed from a polyetherimide resin.

The leads 106 can be configured to direct an electrical stimulus from enclosure 104 to the tissue to which they are connected. In an aspect, the leads 106 can be connected to the circuitry in the enclosure 104. Via the connection to the circuitry, the leads 106 can deliver an electrical impulse generated by the power source to connected tissue. In a further example, the leads 104 can be attached to cardiac tissue to monitor and to generate electrical activity.

In one aspect, the medical device or parts thereof can be formed from a thermoplastic resin. More specifically, the medical device or parts thereof can be formed from polyetherimide resin. In an example, the header 102 can be formed from a polyetherimide. In a further example, the spacers can be formed from a polyetherimide resin.

The patentable scope of the disclosure is defined by the claims, and can include other examples that occur to those skilled in the art. Such other examples are intended to be within the scope of the claims if they have structural elements that do not differ from the literal language of the claims, or if they include equivalent structural elements with insubstantial differences from the literal languages of the claims.

## METHODS

In certain aspects of the disclosure, the medical device or a part thereof can be formed by any method or combination of methods known in the art. These methods include, but are not limited to, molding processes, additive manufacturing, and machining. These molding processes include, but are not limited to, various melt forming process, injection molding, blow molding (stretch, extrusion or injection), sheet and film extrusion, profile extrusion, thermoforming, additive manufacturing, compression molding, fiber extrusion, powder sintering, transfer molding, reaction injection (RIM) molding, vacuum forming, cold casting, dip molding, slush molding and press molding. In one aspect, a combination of these molding methods may be used to form the header or the one or more spacers.

A number of suitable methods may be used to provide a thermoplastic material for the disclosed thermoplastic implant material comprising a thermoplastic resin and a metal coating.

In further aspects, a deposition process such as vapor deposition may be used to deposit a layer of suitable metal at a surface of the thermoplastic resin. Vapor deposition, such as physical vapor deposition, may provide a thin film of a suitable metal at a surface of the implant grade thermoplastic resin. Typically, a suitable metal may be vaporized in a high vacuum environment and may be deposited at the desired surface as a pure metal coating or as an alloy coating.

In further examples, the thermoplastic material according to the present disclosure may be prepared according to an electroplating process to deposit a metal at a surface of the thermoplastic resin. Electroplating solutions typically include the plating metal ion in solution. For example, in U.S. Pat. No. Re. 35,513, one electroplating solution for electroplating of copper includes cuprous chloride, and a solution for plating of silver includes silver chloride. One or more solutions or baths may be used for pretreating and plating, which may be specific to a particular plating metal and/or substrate metal. Multiple baths may be used to coat more than one metal at a thermoplastic substrate. Plating solutions may be used to deposit layers of suitable metals including zirconium and titanium, among others. Metal alloys may be made by providing anodes of different metals. Plating may be performed at moderate temperatures (about 10° C to about 60° C.).

In some examples described herein, the thermoplastic resin may be combined with a conductive filler. The thermoplastic resin may be combined with a conductive additive via extrusion.

In various aspects, a thermoplastic resin as disclosed herein can be prepared according to a variety of methods for use in the medical device. As an example, a polyetherimide resin can be molded to form the header or the spacer by a variety of means such as injection molding, extrusion, rotational molding, blow molding and thermoforming to form the header. The polyetherimide resins of the present disclosure can be blended, compounded, or otherwise combined by a variety of methods involving intimate admixing of the materials with any additional additives desired in the formulation. Because of the availability of melt blending equipment in commercial polymer processing facilities, melt processing methods can be used. In various further aspects, the equipment used in such melt processing methods can include, but is not limited to, the following: co-rotating and counter-rotating extruders, single screw extruders, co-kneaders, disc-pack processors and various other types of extrusion equipment. In a further aspect, the extruder is a twinscrew extruder. In various further aspects, the thermoplastic composition can be processed in an extruder at temperatures from about 180

°C to about 350 °C.

While aspects of the present disclosure can be described and claimed in a particular statutory class, such as the system statutory class, this is for convenience only and one of skill in the art will understand that each aspect of the present invention can be described and claimed in any statutory class. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

Moreover, it is to be understood that unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that an order be inferred, in any respect. This holds for any possible non-express basis for interpretation, including: matters of logic with respect to arrangement of steps or operational flow; plain meaning derived from grammatical organization or punctuation; and the number or type of aspects described in the specification.

## ASPECTS

The present disclosure comprises at least the following aspects.

Aspect 1A: A medical device comprising: an implant grade thermoplastic resin and a metal disposed adjacent a surface of the implant grade thermoplastic resin, wherein at least a portion of the medical device is configured for implantation in tissue.

Aspect 1B: A medical device consisting essentially of: an implant grade thermoplastic resin and a metal disposed adjacent a surface of the implant grade thermoplastic resin, wherein at least a portion of the medical device is configured for implantation in tissue.

Aspect 1C: A medical device consisting of: an implant grade thermoplastic resin and a metal or a conductive additive, wherein at least a portion of the medical device is configured for implantation in tissue.

Aspect 2A: A medical device for implantation in tissue, the medical device comprising: an header comprising one or more bores, wherein the header is formed from an implant grade thermoplastic resin and a metal coating; one or more leads disposed in the header and exiting the header through the one or more bores; and an enclosure coupled to the header at a surface of the enclosure, the enclosure comprising circuitry and a power source.

Aspect 2B: A medical device for implantation in tissue, the medical device consisting essentially of: an header comprising one or more bores, wherein the header is formed from an implant grade thermoplastic resin and a metal coating; one or more leads disposed in the header and exiting the header through the one or more bores; and an enclosure coupled to the header at a surface of the enclosure, the enclosure comprising circuitry and a power source.

Aspect 2C: A medical device for implantation in tissue, the medical device consisting of: an header comprising one or more bores, wherein the header is formed from an implant grade thermoplastic resin and a metal coating; one or more leads disposed in the header and exiting the header through the one or more bores; and an enclosure coupled to the header at a surface of the enclosure, the enclosure comprising circuitry and a power source.

Aspect 3: The medical device of any of claims 1-2, wherein the implant grade thermoplastic resin comprises a polyimide, polysulfone PSU, polyphenylsulfone PPSU, polyether ether ketone PEEK, or polyphthalamide PPA, or a combination thereof.

Aspect 4: The medical device of any of claims 1-3, wherein the implant grade thermoplastic resin comprises a polyetherimide resin comprising structural units derived from at least one diamine selected from 1,3-diaminobenzene, 1,4-diaminobenzene, 4,4'-diaminodiphenyl sulfone, oxydianiline, 1,3-bis(4-aminophenoxy)benzene, or combinations thereof.

Aspect 5: The medical device of any of claims 1-3, wherein the metal or metal coating comprises a transition metal.

Aspect 6: The medical device of any of claims 1-3, wherein the metal or metal coating comprises zirconium or titanium.

Aspect 7: The medical device of any of claims 1-4, further comprising a conductive additive.

Aspect 8: The medical device of claim 7, wherein the conductive additive comprises a conductive carbon.

Aspect 9: The medical device of claim 7, wherein the conductive additive comprises graphene, carbon nanotubes, graphite, or a combination thereof.

Aspect 10: The medical device of any of claims 4-9, wherein the polyetherimide resin has less than 100 ppm amine end groups and a weight average molecular weight of 10,000 to 80,000 Daltons.

Aspect 11: The medical device of any one of claims 4-10, wherein the polyetherimide resin is a fiber polymer having

a diameter of fibers of from about 0.00001 millimeters to about 2 millimeters.

Aspect 12: The medical device of any one of claims 1-10, wherein the thermoplastic resin further comprises a biocide or antimicrobial agent, wherein the biocide is selected from germicides, antimicrobials, antibiotics, antibacterials, anti-yeasts, anti-algals, antivirals, antifungals, antiprotozoals, antiparasites, and combinations thereof.

Aspect 13: The medical device of any one of claims 1-12, wherein the header is formed from a thermoplastic resin comprising between 40 wt% and 90 wt% of a polyetherimide resin and between 10 wt% and 60 wt% of a filler by weight of the thermoplastic resin.

Aspect 14: The medical device of claim 13, wherein the filler comprises glass, carbon, carbon fiber, or a combination thereof.

Aspect 15: The medical device of any of claims 1-14, wherein the implant grade thermoplastic resin and the metal or metal coating cooperate to form the medical device via a process of vapor deposition or electroplating.

Aspect 16: A medical device comprising: a hermetically sealed enclosure; and a header coupled to the enclosure to provide electrical communication to an element within the enclosure, wherein the header is formed from a thermoplastic resin and a metal or metal coating.

Aspect 17: The medical device of claim 16, wherein the thermoplastic resin comprises a polyetherimide resin comprises structural units derived from at least one diamine selected from 1,3-diaminobenzene, 1,4-diaminobenzene, 4,4'-diaminodiphenyl sulfone, oxydianiline, 1,3-bis(4-aminophenoxy)benzene, or combinations thereof.

Aspect 18: The medical device of any of claims 16-17, wherein the metal or metal coating comprises a transition metal.

Aspect 19: The medical device of any of claims 16-18, wherein the metal or metal coating comprises zirconium or titanium.

Aspect 20: The medical device of any of claims 16-19, wherein the conductive additive comprises graphene, carbon nanotubes, graphite, or a combination thereof.

Aspect 21: The medical device of any one of claims 16-20, wherein the thermoplastic resin further comprises a biocide or antimicrobial agent, wherein the biocide is selected from germicides, antimicrobials, antibiotics, antibacterials, anti-yeasts, anti-algals, antivirals, antifungals, antiprotozoals, antiparasites, and combinations thereof.

Aspect 22: The medical device of any of aspects 4-20, wherein the polyetherimide resin has a molecular weight of at least 40,000 Daltons.

Aspect 22A: A sterilized header for an implantable enclosure, the sterilized header comprising a sterilized polymer composition, the polymer composition comprising a) a polyetherimide treated with one of hydrogen peroxide plasma, hydrogen peroxide vapor, or a combination thereof, wherein the sterilized polymer composition further comprises a metal coating.

Aspect 22B: A sterilized header for an implantable enclosure, the sterilized header consisting essentially of a sterilized polymer composition, the polymer composition comprising a polyetherimide treated with one of hydrogen peroxide plasma, hydrogen peroxide vapor, or a combination thereof, wherein the sterilized polymer composition further comprises a metal coating.

Aspect 22C: A sterilized header for an implantable enclosure, the sterilized header consisting of a sterilized polymer composition, the polymer composition comprising a polyetherimide treated with one of hydrogen peroxide plasma, hydrogen peroxide vapor, or a combination thereof, wherein the sterilized polymer composition further comprises a metal coating.

Aspect 23: The sterilized header of aspect any of aspects 22A-22C, wherein the polyetherimide resin comprises structural units derived from at least one diamine selected from 1,3-diaminobenzene, 1,4-diaminobenzene, 4,4'-diaminodiphenyl sulfone, oxydianiline, 1,3-bis(4-aminophenoxy)benzene, or combinations thereof.

Aspect 24: The sterilized header of any one of aspects 22A-23, wherein the polyetherimide has a molecular weight of at least 40,000 Daltons.

Aspect 25: The sterilized header of any one of aspects 22A-24, wherein the polyetherimide has less than 100 ppm amine end groups and a weight average molecular weight of 10,000 to 80,000 Daltons.

Aspect 26: The sterilized header of any one of aspects 22A-25, wherein the polyetherimide resin is a fiber polymer having a diameter of fibers of from about 0.00001 millimeters to about 2 millimeters.

It is also to be understood that the terminology used herein is for the purpose of describing particular aspects only and is not intended to be limiting. As used in the specification and in the claims, the term "comprising" may include the aspects "consisting of' and "consisting essentially of." Unless defined otherwise, all technical and scientific terms used herein have the same meaning as commonly understood by one of ordinary skill in the art to which this disclosure belongs.

As used in the specification and the appended claims, the singular forms "a," "an" and "the" include plural referents unless the context clearly dictates otherwise. Thus, for example, reference to "a polycarbonate" includes mixtures of two or more such polycarbonates. Furthermore, for example, reference to a filler includes mixtures of two or more such fillers.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. A value modified by a term or terms, such as "about" and "substantially," is intended to include the degree of error associated with measurement of the particular quantity based upon the equipment available at the time of filing this application. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "optional" or "optionally" mean that the subsequently described event, condition, component, or circumstance may or may not occur, and that the description includes instances where said event or circumstance occurs and instances where it does not.

As used herein, "implant grade" as in "implant grade thermoplastic" may refer to a material that is suitable for implantation in or at living tissue. I impact grade materials desirably have good strength and corrosion resistance. The criteria for an implant grade rating may be based on a number of applicable ASTM and/or ISO standards, which depend on the type of material. For example, the implant grade thermoplastic resin may qualify as a biocompatible material under ISO 10993/USP6.

Disclosed are component materials to be used to prepare disclosed compositions as well as the compositions themselves to be used within methods disclosed herein. These and other materials are disclosed herein, and it is understood that when combinations, subsets, interactions, groups, etc. of these materials are disclosed that while specific reference of each various individual and collective combinations and permutation of these compounds cannot be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions of the disclosure. Thus, if there are a variety of additional steps that can be performed it is understood that each of these additional steps can be performed with any specific aspect or combination of aspects of the methods of the disclosure.

References in the specification and concluding claims to parts by weight, of a particular element or component in a composition or article denotes the weight relationship between the element or component and any other elements or components in the composition or article for which a part by weight is expressed. Thus, in a composition containing 2 parts by weight of component X and 5 parts by weight component Y, X and Y are present at a weight ratio of 2:5, and are present in such ratio regardless of whether additional components are contained in the compound.

A weight percent of a component, unless specifically stated to the contrary, is based on the total weight of the formulation or composition in which the component is included.

Compounds disclosed herein are described using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group. Unless defined otherwise, technical and scientific terms used herein have the same meaning as is commonly understood by one of skill in the art to which this disclosure belongs.

As used herein, the terms "number average molecular weight" or "Mn" can be used interchangeably, and refer to the statistical average molecular weight of all the polymer chains in the sample and is defined by the formula:

$$Mn = \frac{\sum N_i M_i}{\sum N_i},$$

where $M_i$ is the molecular weight of a chain and $N_i$ is the number of chains of that molecular weight. Mn can be determined for polymers, such as polycarbonate polymers or polycarbonate-polysiloxane copolymers, by methods well known to a person having ordinary skill in the art.

As used herein, the terms "weight average molecular weight" or "Mw" can be used interchangeably, and are defined by the formula:

$$Mw = \frac{\sum N_i M_i^2}{\sum N_i M_i},$$

where Mi is the molecular weight of a chain and Ni is the number of chains of that molecular weight. Compared to Mn, Mw takes into account the molecular weight of a given chain in determining contributions to the molecular weight average. Thus, the greater the molecular weight of a given chain, the more the chain contributes to the Mw. It is to be understood that as used herein, Mw is measured by gel permeation chromatography. In some cases, Mw can be measured by gel permeation chromatography and calibrated with polycarbonate standards. As an example, a polycarbonate of the present disclosure can have a weight average molecular weight of greater than about 5,000 Daltons based on PS standards. As a further example, the polycarbonate can have an Mw of from about 20,000 to about 100,000 Daltons.

## Claims

1. A medical device comprising:
an implant grade thermoplastic resin and a metal disposed adjacent a surface of the implant grade thermoplastic resin, wherein at least a portion of the medical device is configured for implantation in tissue.

2. A medical device for implantation in tissue comprising:

    an header comprising one or more bores, wherein the header is formed from an implant grade thermoplastic resin and a metal coating;
    one or more leads disposed in the header and exiting the header through the one or more bores; and
    an enclosure coupled to the header at a surface of the enclosure, the enclosure comprising circuitry and a power source.

3. The medical device of any of claims 1-2, wherein the implant grade thermoplastic resin comprises a polyimide, polysulfone PSU, polyphenylsulfone PPSU, polyether ether ketone PEEK, or polyphthalamide PPA, or a combination thereof.

4. The medical device of any of claims 1-3, wherein the implant grade thermoplastic resin comprises a polyetherimide resin comprising structural units derived from at least one diamine selected from 1,3-diaminobenzene, 1,4-diaminobenzene, 4,4'-diaminodiphenyl sulfone, oxydianiline, 1,3-bis(4-aminophenoxy)benzene, or combinations thereof.

5. The medical device of any of claims 1-3, wherein the metal or metal coating comprises a transition metal.

6. The medical device of any of claims 1-3, wherein the metal or metal coating comprises zirconium or titanium.

7. The medical device of any of claims 1-4, further comprising a conductive additive.

8. The medical device of claim 7, wherein the conductive additive comprises a conductive carbon.

9. The medical device of claim 7, wherein the conductive additive comprises graphene, carbon nanotubes, graphite, or a combination thereof.

10. The medical device of any of claims 4-9, wherein the polyetherimide resin has less than 100 ppm amine end groups and a weight average molecular weight of 10,000 to 80,000 Daltons.

11. The medical device of any one of claims 4-10, wherein the polyetherimide resin is a fiber polymer having a diameter of fibers of from 0.00001 millimeters to 2 millimeters.

12. The medical device of any one of claims 1-10, wherein the thermoplastic resin further comprises a biocide or antimicrobial agent, wherein the biocide is selected from germicides, antimicrobials, antibiotics, antibacterials, anti-yeasts, anti-algals, antivirals, antifungals, antiprotozoals, antiparasites, and combinations thereof.

**13.** The medical device of any one of claims 1-12, wherein the header is formed from a thermoplastic resin comprising between 40 wt% and 90 wt% of a polyetherimide resin and between 10 wt% and 60 wt% of a filler by weight of the thermoplastic resin.

**14.** The medical device of claim 13, wherein the filler comprises glass, carbon, carbon fiber, or a combination thereof.

**15.** The medical device of any of claims 1-14, wherein the implant grade thermoplastic resin and the metal or metal coating cooperate to form the medical device via a process of vapor deposition or electroplating.

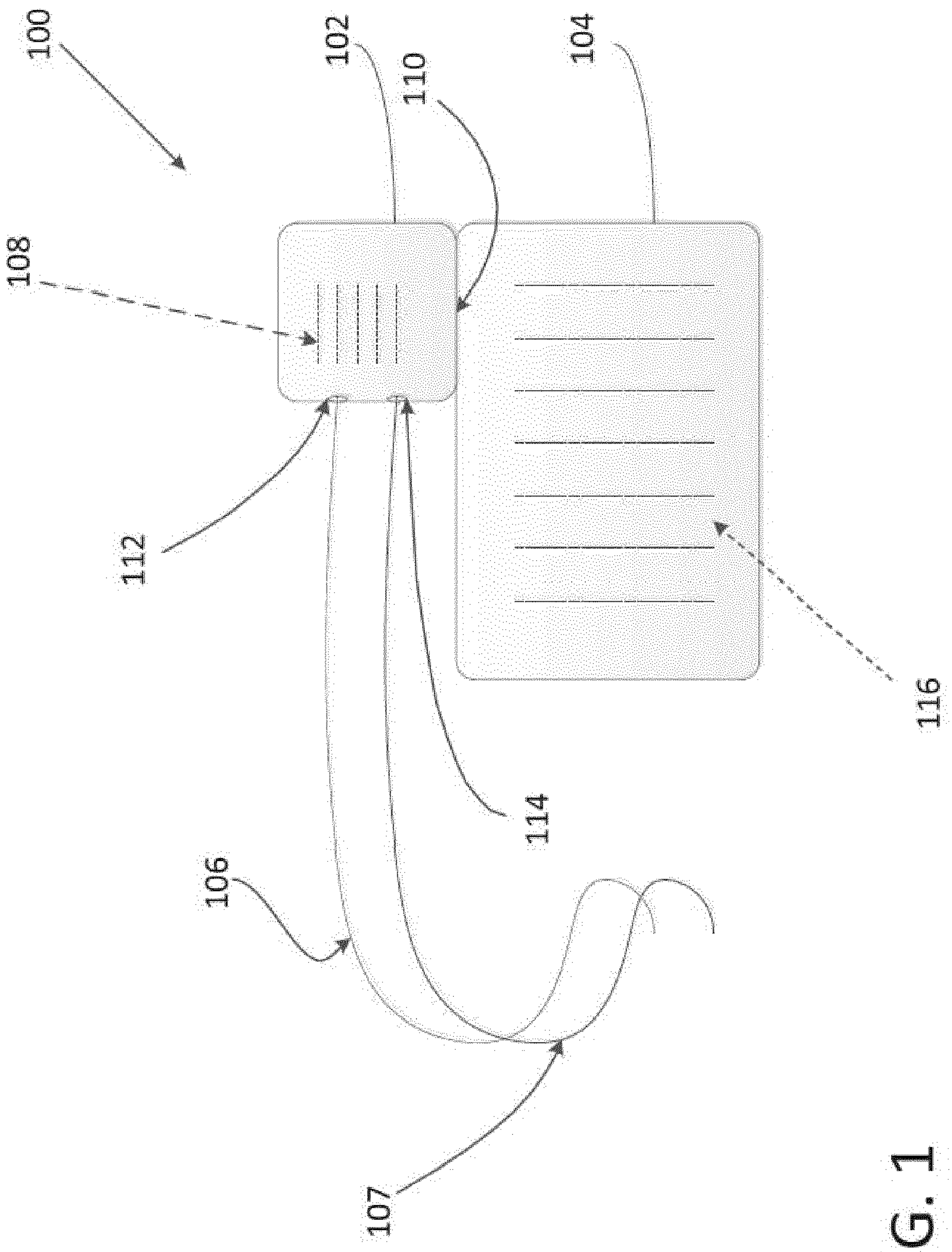


FIG. 1

Europäisches Patentamt
European Patent Office
Office européen des brevets

# EUROPEAN SEARCH REPORT

Application Number
EP 18 18 0068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2016/187424 A1 (SABIC GLOBAL TECHNOLOGIES BV [NL]; KUGLER ANDREW [US]) 24 November 2016 (2016-11-24)<br>* paragraph [0005] - paragraph [0015] *<br>* paragraphs [0017], [0018], [0026], [0029], [0036], [0046] *<br>* paragraphs [0054] - [0056]; claims; examples *<br>----- | 2,4, 10-14<br><br><br>7-9 | INV.<br>A61L27/06<br>A61L27/18<br>A61L27/30<br>A61L27/44<br>A61L27/50<br>A61N1/375<br>A61L31/06<br>A61L31/14 |
| Y<br><br>A | US 2012/025131 A1 (FORERO STEFAN [DE]) 2 February 2012 (2012-02-02)<br>* paragraphs [0001] - [0004] *<br>* paragraphs [0022] - [0026] *<br>* paragraphs [0047], [0052], [0095] - [0097] *<br>* claims; examples *<br>----- | 7-9<br><br>1-4, 10-15 | |
| X<br><br>A | US 2007/237946 A1 (OHRLANDER MATTIAS [SE] ET AL) 11 October 2007 (2007-10-11)<br>* paragraphs [0029], [0030], [0037] *<br>* paragraphs [0038] - [0049] *<br>* claims; examples *<br>----- | 1,3,5,6, 15<br><br>2,4,7-14 | TECHNICAL FIELDS SEARCHED (IPC) |
| A | WO 2016/187454 A1 (SABIC GLOBAL TECHNOLOGIES BV [NL]; KUGLER ANDREW [US]) 24 November 2016 (2016-11-24)<br>* the whole document *<br>----- | 1-15 | A61L<br>A61N |

The present search report has been drawn up for all claims

1

| Place of search | Date of completion of the search | Examiner |
|---|---|---|
| The Hague | 13 December 2018 | Derrien, Anne-Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 18 0068

This annex lists the patent family members relating to the patent documents cited in the above-mentioned European search report. The members are as contained in the European Patent Office EDP file on
The European Patent Office is in no way liable for these particulars which are merely given for the purpose of information.

13-12-2018

| Patent document cited in search report | Publication date | Patent family member(s) | Publication date |
|---|---|---|---|
| WO 2016187424 A1 | 24-11-2016 | US 2018214615 A1 | 02-08-2018 |
| | | WO 2016187424 A1 | 24-11-2016 |
| US 2012025131 A1 | 02-02-2012 | CN 102317360 A | 11-01-2012 |
| | | CN 106167602 A | 30-11-2016 |
| | | EP 2373726 A1 | 12-10-2011 |
| | | JP 2012511799 A | 24-05-2012 |
| | | JP 2015165501 A | 17-09-2015 |
| | | KR 20110111401 A | 11-10-2011 |
| | | US 2012025131 A1 | 02-02-2012 |
| | | WO 2010066730 A1 | 17-06-2010 |
| US 2007237946 A1 | 11-10-2007 | AR 061594 A1 | 10-09-2008 |
| | | AT 539773 T | 15-01-2012 |
| | | AU 2007235714 A1 | 18-10-2007 |
| | | BR PI0710600 A2 | 16-08-2011 |
| | | CA 2647606 A1 | 18-10-2007 |
| | | CN 101184512 A | 21-05-2008 |
| | | CN 101460200 A | 17-06-2009 |
| | | CN 101478994 A | 08-07-2009 |
| | | DK 2012839 T3 | 10-04-2012 |
| | | EP 2007440 A1 | 31-12-2008 |
| | | EP 2010229 A1 | 07-01-2009 |
| | | EP 2012839 A1 | 14-01-2009 |
| | | ES 2379989 T3 | 07-05-2012 |
| | | ES 2447466 T3 | 12-03-2014 |
| | | ES 2568681 T3 | 03-05-2016 |
| | | IL 194436 A | 30-11-2015 |
| | | JP 5619416 B2 | 05-11-2014 |
| | | JP 5806698 B2 | 10-11-2015 |
| | | JP 5881773 B2 | 09-03-2016 |
| | | JP 5881774 B2 | 09-03-2016 |
| | | JP 2009533081 A | 17-09-2009 |
| | | JP 2009536043 A | 08-10-2009 |
| | | JP 2009536837 A | 22-10-2009 |
| | | JP 2013208431 A | 10-10-2013 |
| | | JP 2014221273 A | 27-11-2014 |
| | | JP 2014236988 A | 18-12-2014 |
| | | KR 20090031668 A | 27-03-2009 |
| | | MY 147571 A | 31-12-2012 |
| | | PT 2012839 E | 18-04-2012 |
| | | RU 2008139083 A | 20-05-2010 |
| | | SI 2012839 T1 | 31-08-2012 |
| | | TW 200744686 A | 16-12-2007 |
| | | US 2007237945 A1 | 11-10-2007 |
| | | US 2007237946 A1 | 11-10-2007 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 18 18 0068

This annex lists the patent family members relating to the patent documents cited in the above-mentioned European search report. The members are as contained in the European Patent Office EDP file on
The European Patent Office is in no way liable for these particulars which are merely given for the purpose of information.

13-12-2018

| Patent document cited in search report | Publication date | Patent family member(s) | Publication date |
| --- | --- | --- | --- |
| | | US 2009123733 A1 | 14-05-2009 |
| | | US 2011236441 A1 | 29-09-2011 |
| | | US 2013115262 A1 | 09-05-2013 |
| | | US 2014141052 A1 | 22-05-2014 |
| | | US 2016250390 A1 | 01-09-2016 |
| | | US 2018104385 A1 | 19-04-2018 |
| | | WO 2007117191 A1 | 18-10-2007 |
| | | WO 2007117213 A1 | 18-10-2007 |
| | | WO 2007117214 A1 | 18-10-2007 |
| | | ZA 200808504 B | 29-07-2009 |
| WO 2016187454 A1 | 24-11-2016 | US 2018126047 A1 | 10-05-2018 |
| | | WO 2016187454 A1 | 24-11-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5229482 A
- US 3875116 A
- US 6919422 B
- US 6355723 A
- US 4690997 A
- US 4808686 A
- US 7041773 B
- US RE35513 E


### Non-patent literature cited in the description


- *CHEMICAL ABSTRACTS,* 148935-94-8